# EUROPEAN PATENT SPECIFICATION

(11) **EP 1 697 533 B1**
(45) Date of publication and mention of the grant of the patent: **01.12.2010**
(21) Application number: 04817937.8
(22) Date of filing: 03.12.2004
(51) Int. Cl.: C12Q 1/68

(54) **Method for assessing the relative genetic risk for autism**
Verfahren zur Bestimmung des relativen genetischen Risikos für Autismus
Procédé pour déterminer le risque génétique relatif à l'autisme

(30) Priority: 05.12.2003 US 527630 P
(43) Date of publication of application: 06.09.2006
(73) Proprietor: Beatrice and Samuel A. Seaver Foundation, New York, NY 10022 (US)
(72) Inventor: BUXBAUM, Joseph D., New York, NY 10025 (US); RAMOZ, Nicolas, New York, NY 10029 (US)
(74) Representative: Chajmowicz, Marion
(86) International application number: PCT/US2004/040444
(87) International publication number: WO 2005/055807

(56) References cited:
- LASORSA FRANCESCO MASSIMO ET AL: "Recombinant expression of the Ca(2+)-sensitive aspartate/glutamate carrier increases mitochondrial ATP production in agonist-stimulated Chinese hamsterovary cells" JOURNAL OF BIOLOGICAL CHEMISTRY, THE AMERICAN SOCIETY OF BIOLOGICAL CHEMISTS, INC.,, US, vol. 278, no. 40, 8 July 2003 (2003-07-08), pages 38686-38692, XP009093294 ISSN: 0021-9258
- "SNP Information for IMS-JST045724" INTERNET CITATION, [Online] 26 March 2001 (2001-03-26), XP007903644 Retrieved from the Internet: URL:http://snp.ims.u-tokyo.ac.jp/cgi-bin/S npInfo.cgi?SNP_ID=IMS-JST045724> [retrieved on 2007-12-05]
- NCBI: "SINGLE NUCLEOTIDE POLYMORPHISM" INTERNET CITATION, 29 January 2001 (2001-01-29), XP007903650 [retrieved on 2007-12-05]
- BUXBAUM J D ET AL: "Association between a GABRB3 polymorphism and autism" MOLECULAR PSYCHIATRY, BASINGSTOKE, GB, vol. 7, no. 3, 2002, pages 311-316, XP009093355 ISSN: 1359-4184
- BACCHELLI E ET AL: "Screening of nine candidate genes for autism on chromosome 2q reveals rare nonsynonymous variants in the cAMP-GEFII gene" MOLECULAR PSYCHIATRY, BASINGSTOKE, GB, vol. 8, no. 11, November 2003 (2003-11), pages 916-924, XP009093364 ISSN: 1359-4184
- BUXBAUM J D ET AL: "Evidence for a susceptibility gene for autism on chromosome 2 and for genetic heterogeneity" AMERICAN JOURNAL OF HUMAN GENETICS, AMERICAN SOCIETY OF HUMAN GENETICS, CHICAGO, IL, US, vol. 68, no. 6, June 2001 (2001-06), pages 1514-1520, XP009093556 ISSN: 0002-9297
- PHILIPPE ANNE ET AL: "Analysis of ten candidate genes in autism by association and linkage" AMERICAN JOURNAL OF MEDICAL GENETICS, WILEY, NEW YORK,NY, US, vol. 114, no. 2, 8 March 2002 (2002-03-08), pages 125-128, XP009093372 ISSN: 0148-7299
- JAMAIN S ET AL: "LINKAGE AND ASSOCIATION OF THE GLUTAMATE RECEPTOR 6 GENE WITH AUTISM" MOLECULAR PSYCHIATRY, BASINGSTOKE, GB, vol. 7, no. 3, 2002, pages 302-310, XP008028608 ISSN: 1359-4184
- GIBSON U E M ET AL: "A NOVEL METHOD FOR REAL TIME QUANTITATIVE RT-PCR" GENOME RESEARCH, COLD SPRING HARBOR LABORATORY PRESS, WOODBURY, NY, US, vol. 6, no. 10, October 1996 (1996-10), pages 995-1001, XP000642796 ISSN: 1088-9051
- DATABASE EMBL [Online] 26 July 2002 (2002-07-26), "Homo sapiens mRNA for mitochondrial aspartate-glutamate carrier protein (SLC25A12 gene)" XP007903639 retrieved from EBI accession no. EMBL:AJ496568 Database accession no. AJ496568
- RAMOZ N ET AL: "Linkage and Association of the Mitochondrial Aspartate/Glutamate Carrier SLC25A12 Gene With Autism" AMERICAN JOURNAL OF PSYCHIATRY, AMERICAN PSYCHIATRIC ASSOCIATION, WASHINGTON, DC, US, vol. 161, no. 4, April 2004 (2004-04), pages 662-669, XP002996520 ISSN: 0002-953X
- LUCENTINI J.: 'Gene Association Studies Typically Wrong.' vol. 18, no. 24, 20 December 2004, pages 1 - 3, XP002994836
- KROESE M ET AL: 'Genetic tests and their evaluation: can we answer the key questions?' GENET MED. vol. 6, no. 6, November 2004 - December 2004, pages 475 - 480, XP008061434
- VEENSTRA-VANDER WEELE J.: 'Autism as a Paradigmatic Complex Genetic Disorder.' ANNU REV GENOMICS HUM GENET. vol. 5, 2004, pages 379 - 405, XP002996518
- SAHEKI T ET AL: 'Mitochondrial aspartate glutamate carrier (citrin) deficiency as the cause of adult-onset type II citrullinemia (CTLN2) and idiopathic neonatal hepatitis (NICCD).' J HUMAN GENET. vol. 47, 2002, pages 333 - 341, XP002996519
- SANZ R ET AL: 'Assignment of the calcium-binding mitochondrial carrier Aralar1 gene (SLC25A12) to Human Chromosome Band 2q31 by in Situ Hybridization.' CYTOGENET CELL GENET. vol. 89, no. 3/4, 2000, pages 143 - 144, XP008062687
- RAMOZ N ET AL: 'Linkage and Association of the Mitochondrial Aspartate/Glutamate Carrier SLC25A12 Gene With Autism.' AM J PSYCHIATRY. vol. 161, April 2004, pages 662 - 669, XP002996520

## Description

### STATEMENT REGARDING FEDERALLY SPONSORED RESEARCH OR DEVELOPMENT

The U.S. Government has a paid-up license in this invention and the right in limited circumstances to require the patent owner to license others on reasonable terms as provided by the terms of Grant No. U54 MH066673 awarded by the National Institutes of Health.

### BACKGROUND OF THE INVENTION

### (1) Field of the Invention

The present invention generally relates to autism risk assessment. More specifically, methods and compositions are provided that are useful for estimating risk for having autism.

### (2) Description of the Related Art

References cited:
1. Lord C, Leventhal BL, Cook EH Jr: Quantifying the phenotype in autism spectrum disorders. Am J Med Genet 2001; 105:36-38.
2. Lord C, Cook EH, Leventhal BL, Amaral DG: Autism spectrum disorders. Neuron 2000; 28:355-63.
3. Rapin I, Katzman R: Neurobiology of autism. Ann Neurol 1998; 43:7-14.
4. Folstein SE, Rosen-Sheidley B: Genetics of autism: complex aetiology for a heterogeneous disorder. Nat Rev Genet 2001; 2:943-955.
5. Rutter M: Concepts of autism: a review of research. J Child Psychol Psychiatry 1968; 9:1-25.
6. Bailey A, Le Couteur A, Gottesman 1, Bolton P, Simonoff E, Yuzda E, Rutter M: Autism as a strongly genetic disorder: evidence from a British twin study. Psychol Med 1995; 25:63-77.
7. Lucinio J and Alvarado J: Progress in the genetics of autism. Mol Psychiatry 2002; 7:1012-1017.
8. Philippe A, Guilloud-Bataille M, Martinez M, Gillberg C, Rastam M, Sponheim E, Coleman M, Zappella M, Aschauer H, Penet C, Feingold J, Brice A, Leboyer M: Analysis of ten candidate genes in autism by association and linkage. Am J Med Genet 2002; 114:125-128.
9. Buxbaum JD, Silverman JM, Smith CJ, Greenberg DA, Kilifarski M, Reichert J, Cook EH Jr, Fang F, Song C-Y, Vitale R: Association between a GABRB3 polymorphisms and autism. Mol Psych 2002; 7:311-316.
10. Jamain S, Betancur C, Quach H, Philippe A, Fellous M, Giros B, Gillberg C, Leboyer M, Bourgeron T: Linkage and association of the glutamate receptor 6 gene with autism. Mol Psychiatry 2002; 7:302-310.
11. Kim SJ, Cox N, Courchesne R, Lord C, Corsello C, Akshoomoff N, Guter S, Leventhal BL, Courchesne E, Cook EH Jr: Transmission disequilibrium mapping at the serotonin transporter gene (SLC6A4) region in autistic disorder. Mol Psychiatry 2002; 7:278-288.
12. Veenstra-Vanderweele J, Cook E Jr, Lombroso PJ: Genetics of childhood disorders: XLVI. Autism, part 5: genetics of autism. J Am Acad Child Adolesc Psychiatry 2003; 42:116-118.
13. Buxbaum JD, Silverman JM, Smith CJ, Kilifarski M, Reichert J, Hollander E, Lawlor BA, Fitzgerald M, Greenberg DA, Davis KL: Evidence for a susceptibility gene for autism on chromosome 2 and for genetic heterogeneity. Am J Hum Genet 2001; 68:1514-1520.
14. International Molecular Genetic Study of Autism Consortium (IMGSAC): A genomewide screen for autism: strong evidence for linkage to chromosomes 2q, 7q, and 16p. Am J Hum Genet 2001; 69:570-581.
15. Shao Y, Raiford KL, Wolpert CM, Cope HA, Ravan SA, Ashley-Koch AA, Abramson RK, Wright HH, DeLong RG, Gilbert JR, Cuccaro ML, Pericak-Vance MA: Phenotypic homogeneity provides increased support for linkage on chromosome 2 in autistic disorder. Am J Hum Genet 2002; 70:1058-1061.
16. Bacchelli E, Blasi F, Biondolillo M, Lamb JA, Bonora E, Barnby G, Parr J, Beyer KS, Klauck SM, Poustka A, Bailey AJ, Monaco AP, Maestrini E: Screening of nine candidate genes for autism on chromosome 2q reveals rare nonsynonymous variants in the cAMP-GEFII gene; Mol Psychiatry 2003; 8:916-24.
17. Weiss LA, Escayg A, Kearney JA, Trudeau M, MacDonald BT, Mori M, Reichert J, The AGRE Consortium, Buxbaum JD, Meisler MH: Sodium channels SCN1A, SCN2A and SCN3A in familial autism. Mol Psychiatry 2002; 8:182-190.
18. Geschwind DH, Sowinski J, Lord C, Iversen P, Shestack J, Jones P, Ducat L, Spence SJ: The autism genetic resource exchange: a resource for the study of autism and related neuropsychiatric conditions. Am J Hum Genet 2001; 69:463-466.
19. Lord C, Rutter M, Le Couteur A: Autism Diagnostic Interview-Revised: a revised version of a diagnostic interview for caregivers of individuals with possible pervasive developmental disorders. J Autism Dev Disord. 1994; 24:659-685.
20. Olivier M, Chuang LM, Chang MS, Chen YT, Pei D, Ranade K, de Witte A, Allen J, Tran N, Curb D, Pratt R, Neefs H, de Arruda Indig M, Law S, Neri B, Wang L, Cox DR: High-throughput genotyping of single nucleotide polymorphisms using new biplex invader technology. Nucleic Acids Res 2002; 30:e53.
21. Spielman RS, Ewens WJ: A sibship test for linkage in the presence of association: the sib transmission/disequilibrium test. Am J Hum Genet 1998; 62:450-458.
22. Durner M, Vieland VJ, Greenberg DA: Further evidence for the increased power of LOD scores compared with nonparametric methods. Am J Hum Genet 1999; 64:281-289.
23. Hodge SE, Vieland VJ, Greenberg DA: HLODs remain powerful tools for detection of linkage in the presence of genetic heterogeneity. Am J Hum Genet 2002; 70:556-559
24. Xie X, Ott J: Testing linkage disequilibrium between a disease gene and marker loci. Am J Hum Genet 1993; 53:(Suppl) 110.
25. del Arco A, Satrustegui J: Molecular cloning of Aralar, a new member of the mitochondrial carrier superfamily that binds calcium and is present in human muscle and brain. J Biol Chem 1998; 273:23327-23334.
26. Palmieri L; Pardo B, Lasorsa FM, del Arco A, Kobayashi K, Iijima M, Runswick MJ, Walker JE, Saheki T, Satrustegui J, Palmieri F: Citrin and aralar1 are Ca(2+)-stimulated aspartate/glutamate transporters in mitochondria. EMBO J 2001; 20:5060-5069.
27. Ramos M, del Arco A, Pardo B, Martinez-Serrano A, Martinez-Morales JR, Kobayashi K, Yasuda T, Bogonez E, Bovolenta P, Saheki T, Satrustegui J: Developmental changes in the Ca2+-regulated mitochondrial aspartate-glutamate carrier aralar1 in brain and prominent expression in the spinal cord. Dev Br Research 2003; 143:33-46.
28. Filipek PA, Juranek J, Smith M, Mays LZ, Ramos ER, Bocian M, Masser-Frye D, Laulhere TM, Modahl C, Spence MA, Gargus JJ: Mitochondrial dysfunction in autistic patients with 15q inverted duplication. Ann Neurol 2003; 53:801-804.
29. Badner JA, Gershon ED: Regional meta-analysis of published data supports linkage of autism with markers on chromosome 7. Mol Psychiatry 2002; 7:56-66.
30. Botstein D, Risch N: Discovering genotypes underlying human phenotypes: past successes for mendelian disease, future approaches for complex disease. Nat Genet 2003; 33:(Suppl)228-237.
31. Hugot JP, Chamaillard M, Zouali H, Lesage S, Cezard J-P, Belaiche J, Almer S, Tysk C, O'Morain CA, Gassull M, Binder V, Finkel Y, Cortot A, Modigliani R, Laurent-Puig P, Gower-Rousseau C, Macry J, Colombel J-F, Sahbatou M, Thomas G: Association of NOD2 leucine-rich repeat variants with susceptibility to Crohn's disease. Nature 2001; 411:599-603.
32. Horikawa Y, Oda N, Cox NJ, Li X, Orho-Melander M, Hara M, Hinokio Y, Lindner TH, Mashima H, Schwarz PE, del Bosque-Plata L, Horikawa Y, Oda Y, Yoshiuchi I, Colilla S, Polonsky KS, Wei S, Concannon P, Iwasaki N, Schulze J, Baier LJ, Bogardus C, Groop L, Boerwinkle E, Hanis CL, Bell GI: Genetic variation in the gene encoding calpain-10 is associated with type 2 diabetes mellitus. Nat Genet 2000; 26:163-75.
33. Yan H, Yuan W, Velculescu VE, Vogelstein B, Kinzler KW: Allelic variation in human gene expression. Science 2002; 297:1143-5
34. Bray NJ, Buckland PR, Owen MJ, ODonovan MC: Cis-acting variation in the expression of a high proportion of genes in human brain. Hum Genet 2003; 149-53.
35. Lasorsa FM, Pinton P, Palmieri L, Fiermonte G, Rizzuto R, Palmieri F: Recombinant expression of the Ca(2+)-sensitive aspartate/glutamate carrier increases mitochondrial ATP production in agonist-stimulated Chinese hamster ovary cells. J Biol Chem 2003; 278:38686-92.
36. Nyholt DR. All LODs are not created equal. Am J Hum Genet 200:282-288.

- D1:: LASORSA FRANCESCO MASSIMO ET AL: "Recombinant expression of the Ca(2+)-sensitive aspartate/glutamate carrier increases mitochondrial ATP production in agonist-stimulated Chinese hamsterovary cells" JOURNAL OF BIOLOGICAL CHEMISTRY, THE AMERICAN SOCIETY OF BIOLOGICAL CHEMISTS, INC." US, vol. 278, no. 40, 8 July 2003 (2003-07-08), pages 38686-38692, XP009093294 ISSN: 0021-9258
- D2:: "SNP Information for IMS-JST045724" INTERNET CITATION, [Online] 26 March 2001 (2001-03-26), XP007903644 Retrieved from the Internet: URL:http://snp.ims.u-tokyo.ac.jp/cgi-bin/S nplnfo.cgi?SNP_ID=IMS-JST045724> [retrieved on 2007-12-05]
- D3:: NCBI: "SINGLE NUCLEOTIDE POLYMORPHISM" INTERNET CITATION, 29 January 2001 (2001-01-29), XP007903650
- D4:: BUXBAUM J D ET AL: "Association between a GABRB3 polymorphism and autism" MOLECULAR PSYCHIATRY, BASINGSTOKE, GB, vol. 7, no. 3, 2002, pages 311-316, XP009093355 ISSN: 1359-4184
- D5:: BACCHELLI E ET AL: "Screening of nine candidate genes for autism on chromosome 2q reveals rare nonsynonymous variants in the cAMP-GEFII gene" MOLECULAR PSYCHIATRY, BASINGSTOKE, GB, vol. 8, no. 11, November 2003 (2003-11), pages 916-924, XP009093364 ISSN: 1359-4184
- D6:: BUXBAUM J D ET AL: "Evidence for a susceptibility gene for autism on chromosome 2 and for genetic heterogeneity" AMERICAN JOURNAL OF HUMAN GENETICS, AMERICAN SOCIETY OF HUMAN GENETICS, CHICAGO, IL, US, vol. 68, no. 6, June 2001 (2001-06), pages 1514-1520, XP009093556 ISSN: 0002-9297
- D7:: PHILIPPE ANNE ET AL: "Analysis of ten candidate genes in autism by association and linkage" AMERICAN JOURNAL OF MEDICAL GENETICS, WILEY, NEW YORK,NY, US, vol. 114, no. 2, 8 March 2002 (2002-03-08), pages 125-128, XP009093372 ISSN: 0148-7299
- D8:: JAMAIN S ET AL: "LiNKAGE AND ASSOCIATION OF THE GLUTAMATE RECEPTOR 6 GENE WITH AUTISM" MOLECULAR PSYCHIATRY, BASINGSTOKE, GB, vol. 7, no. 3, 2002, pages 302-310, XP008028608 ISSN: 1359-4184
- D9:: GIBSON U E M ET AL: "A NOVEL METHOD FOR REAL TIME QUANTITATIVE RT-PCR" GENOME RESEARCH, COLD SPRING HARBOR LABORATORY PRESS, WOODBURY, NY, US, vol. 6, no. 10, October 1996 (1996-10), pages 995-1001, XP000642796 ISSN: 1088-9051
- D10:: DATABASE EMBL [Online] 26 July 2002 (2002-07-26), "Homo sapiens mRNA for mitochondrial aspartate-glutamate carrier protein (SLC25A12 gene)" XP007903639 retrieved from EBI accession no. EMBL:AJ496568 Database accession no. AJ496568

Autism or autistic disorder (MIM #209850) is a neurodevelopmental disorder characterized by a deficit in verbal and non-verbal communication, impairments in reciprocal social interactions, and patterns of repetitive or stereotyped behaviors and interests (1-3). The sex-ratio is 4:1 male to female, and the prevalence of the disease is currently thought to possibly be above 1 per 1000 persons (4). Autism appears to be the most highly genetic of the psychiatric disorders as evidenced by the high risk of autism in additional children in families with an autistic child (estimated to be 50 to 100 times greater than that expected by chance) and the concordance rate for monozygotic twins being much higher than that of dizygotic twins (5). Heritability estimates of idiopathic autism are above 90% (6), so much of the disorder can be attributed to a genetic etiology. However, autism does not follow a simple Mendelian mode of transmission (i.e., dominant or recessive transmission) but is clearly a polygenic disease (4). A commonly accepted genetic model involves several genes (between 5 to 10) that interact to produce the disorder.

A genetic mutation or variant segregating with autism has yet to be unequivocally identified. Candidate genes for studies of autism range from genes that are thought to play a role in neurodevelopmental pathways, comportment or behavior, such as genes in the serotonergic pathway or reelin (4, 7, 8). A few polymorphisms in several genes have been associated with the disorder in certain studies, but not in others (4, 9-11).

Several independent studies involving genome-wide scans have now been published and point to significant linkage between autism and chromosome 2q and 7q regions (4,12). Our studies defined chromosome 2q24-q33 as a susceptibility region for autism with a peak at D2S335, particularly evident in families with more severe autism [as defined by delayed onset (over 36 months) of phrase speech (phrase speech delay, PSD) (MIM #606053)] (NPL score of 3.32 and a HLOD of 2.99) (13). Using a cohort of 152 autism sibling-pair families mostly from European countries, the International Molecular Genetic Study of Autism Consortium (IMGSAC) reported their highest multipoint LOD score (MLS=3.74) at D2S2188 in families with autism with language delay (defined in that study as no single word before 24 months and/or no phrase speech before 33 months) (14). When stricter diagnostic criteria were used, the MLS increased to a value of 4.8. Finally, a study from the Collaborative Autism Team using the PSD criteria to weight its data also showed a linkage between autism and chromosome region 2q33, with an MLS of 2.86 and a HLOD of 2.12 at D2S 116 (15).

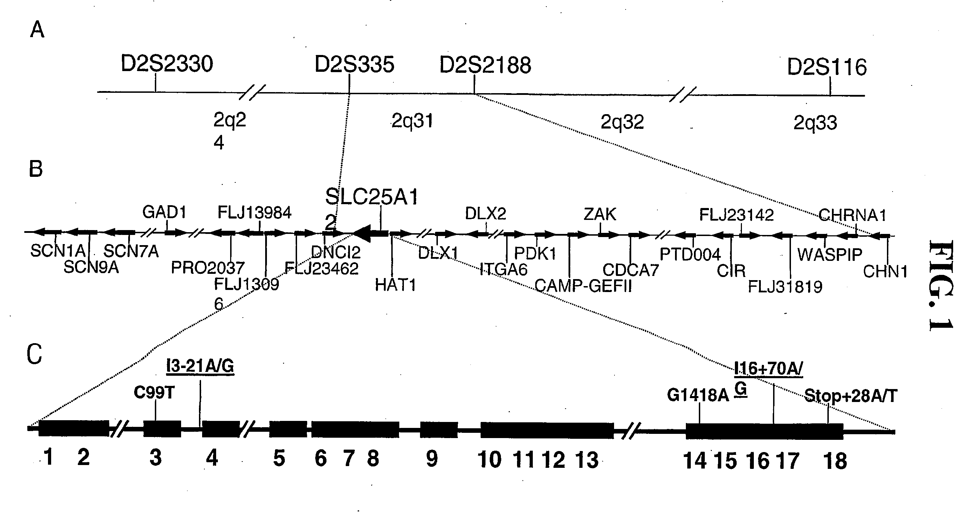

D2S335, D2S2188 and D2S 116 are localized on chromosome 2 at 171 megabases (Mb), 174.4 Mb and 200.5 Mb, respectively (Figure 1A). This indicates that a critical region of susceptibility for autism occurs near D2S335 and D2S2188 in 2q31. In this interval, several known genes and expressed sequence tags have been mapped (Figure 1B). Recently, the IMGSAC has reported the analysis of nine candidate genes (TBR1, GAD1, DLX1, DLX2, cAMP-GEFII, CHN1, CREB2, HOXD1 and NEUROD1) localized across a 30 Mb region of 2q, that are expressed in the central nervous system and encode proteins that play a role in neuronal cells or in neurobiological pathways (16). Variants were observed in TBR1, cAMP-GEFII, CHN1, HOXD1 and NEUROD1. However, no evidence was found that any of the candidate genes contributes to autism. In this region, our laboratory, together with the laboratory of Dr. Miriam Meisler, has previously investigated the neuronal voltage-gated sodium channels type I, II and III (SCN1A, SCN2A and SCN3A) in 117 multiplex autism families (17). Rare mutations were identified, each in single families, that were not observed in controls. These mutations, while of great interest, are not likely to account for the evidence of linkage observed in this region.

There is thus a need to pinpoint genetic variations associated with autism, in order to be able to determine whether individuals are at particular risk for autism, and to determine the risk for autism in offspring of two individuals. Identification of genetic risk factors would also provide tools and information for elucidating the causes of autism. The present invention addresses that need.

### SUMMARY OF THE INVENTION

Accordingly, the inventors have discovered that the risk of autism is increased in individuals having the G allele at either or both polymorphism sites rs2056202 and rs2292813 of the SLC25A12 gene.

Thus, in some embodiments, the invention is directed to method of evaluating an individual for relative genetic risk for autism. The methods comprise determining the individual's genotype at polymorphism sites rs2056202 and/or rs2292813 of the SLC25A12 gene. In these embodiments, the presence of a G at either of the two sites indicates an increased risk for autism, and the presence of an increasing number of G's at the sites indicates an increasing risk for autism.

The invention discloses sets of two primers suitable for use in polymerase chain reaction, useful for the methods identified immediately above. The invention additionally discloses to kits comprising the above-identified primers.

In other embodiments, the invention discloses polynucleotides consisting of any of the sequences of SEQ ID NO:1, SEQ ID NO:2, SEQ ID NO:3, and SEQ ID NO:4.

The invention further discloses methods of identifying a form of a genetic polymorphism that is linked to autism. The methods comprise identifying a polymorphism in the SLC25A12 gene and determining whether one form of the polymorphism is present in autistic individuals more than another form. In these embodiments, in the form that is present more often in autism is linked to autism. The polymorphisms identified by these methods can also be used to determine the risk of an individual to autism.

In further embodiments, the invention discloses eukaryotic cells comprising a transgenic human SLC25A12 gene, and non-human animals comprising those cells.

Additionally, the invention discloses methods of evaluating whether a compound affects autism. The methods comprise contacting the compound with the above described eukaryotic cell, then determining whether the compound affects expression or activity of a product of the SLC25A12 gene. In these embodiments, a compound that affects expression or activity of the product of the SLC25A12 gene affects autism.

### BRIEF DESCRIPTION OF THE DRAWINGS

FIG. 1 is three graphics showing the genomic organization of the autism susceptibility locus on chromosome region 2q24-q33. Panel A shows the genetic and cytogenetic mapping. Panel B shows the organization of positional candidate genes. Arrowhead indicates orientation of transcription. Panel C shows the genomic structure of SLC25A12 gene. Variants identified in the present study are indicated (see text), with the two SNPS focused on in the current study, rs2056202 (I3-21A/G) and rs2292813 (I16+70A/G) underlined.

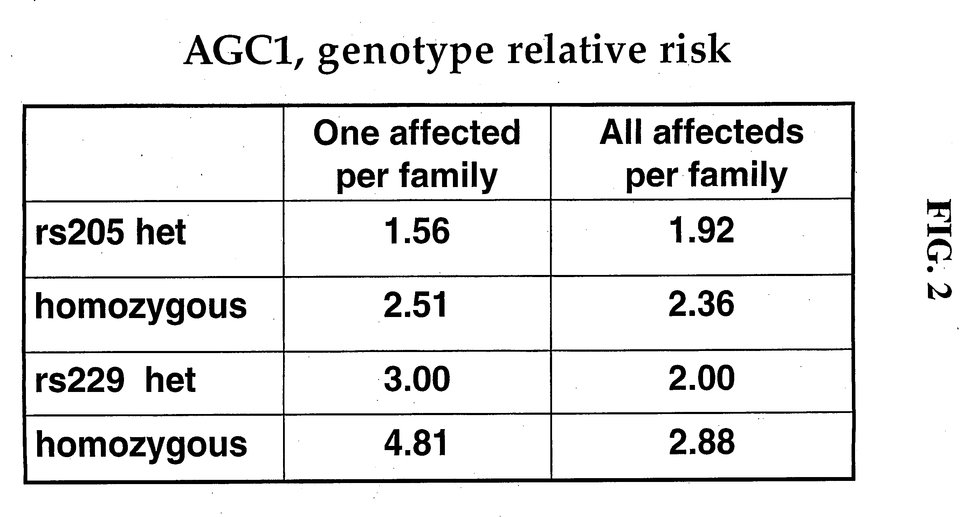

FIG. 2 is a table showing the results of a relative risk assessment using polymorphisms of the SLC25A12 gene.

### DETAILED DESCRIPTION OF THE INVENTION

The present invention is based on the discovery of an association between certain polymorphisms in the SLC25A12 gene and autism. This discovery enables various compositions and methods for use in various aspects of autism diagnostics, therapeutics, and research.

Thus, in some embodiments, the invention is directed to method of evaluating an individual for relative genetic risk for autism. The methods comprise determining the in dividual's genotype at polymorphism sites rs2056202 and/or rs2292813 of the SLC25A12 gene. In these embodiments, the presence of a G at either of the two sites indicates an increased risk for autism, and the presence of an increasing number of G's at the sites indicates an increasing risk for autism.

Any human can be tested using these embodiments, and these methods can also be used to determine the potential risk of the offspring of two individuals for autism, based on the identified genotype of the parents at the relevant polymorphism.

The invention is not narrowly limited to any particular method for determining the individual's genotype at the relevant polymorphic sites; the skilled artisan could chose and apply an appropriate method for any particular application without undue experimentation. In some preferred embodiments, the genotype is determined by single strand conformation polymorphism, denaturing high-performance liquid chromatography, DNA Invader, and/or polymerase chain reaction amplification followed by sequencing. See, e.g., Example 1.

In those embodiments employing polymerase chain reaction (PCR), preferred primers for amplifying the appropriate regions are comprise SEQ ID NO:5 and SEQ ID NO:6 (for amplifying the relevant region of rs2056202 [these primers amplify SEQ ID NO:1 or SEQ ID NO:2, depending on the polymorphic form present]) or SEQ ID NO:7 and SEQ ID NO:8 (for amplifying the relevant region of rs2292813 [amplifying SEQ ID NO:3 or SEQ ID NO:4]).

The invention describes sets of two primers suitable for use in polymerase chain reaction, useful for the methods of determining an individual's risk for autism, as described above. Preferred primer sets are SEQ ID NO:5 and SEQ ID NO:6, for amplifying the relevant region of rs2056202. and SEQ ID NO:7 and SEQ ID NO:8, for amplifying the relevant region of rs2292813. However, any other primers that are specific for either of the relevant regions of the SLC25A12 gene could also be used, and can be designed without undue experimentaion.

In additional embodiments, the invention describes kits comprising at least one set of primers suitable for use in polymerase chain reaction (PCR). The set of primers in these kits amplifies polymorphism site rs2056202 or rs2292813, or both of the SLC25A12 gene. Consistent with the above discussion describing the relevant primers, preferred primer sets are SEQ ID NO:5 and SEQ ID NO:6, for amplifying the relevant region of rs2056202, and SEQ ID NO:7 and SEQ ID NO:8, for amplifying the relevant region of rs2292813. However, any other primers that are specific for either of the relevant regions of the SLC25A12 gene could also be used. These kits can also comprise instructions for using the set(s) of primers to evaluate an individual for relative genetic risk for autism by determining the genotype of the polymorphic sites re2056202 and/or re2292813 of the SLC25A12 gene. The kits can also comprise other ingredients such as buffers, enzymes and the like, for performing PCR and/or analysis of the PCR product(s).

The invention additionally disloses PCR products amplified using any of the above-described primer sets. Nonlimiting examples of these PCR products include SEQ ID NO:1, SEQ ID NO:2, SEQ ID NO:3, and SEQ ID NO:4.

The invention further describes methods of identifying a form of a genetic polymorphism that is linked to autism. The methods comprise identifying a polymorphism in the SLC25A12 gene and determining whether one form of the polymorphism is present in autistic individuals more than another form. In these embodiments, in the form that is present more often in autism is linked to autism. The polymorphisms identified by these methods can also be used to determine the risk of an individual to autism. Nonlimiting examples of procedures to conduct these methods are provided in the Example 1. Genetic polymorphisms identified by the above procedure that are associated with autism can also be used to evaluate an individual for relative genetic risk for autism.

Since this invention includes the discovery that the SLC25A12 gene is associated with autism, the skilled artisan would understand that cells and multicellular organisms comprising the human SLC25A12 gene are useful for autism research.

Thus, the instant invention also discloses eukaryotic cells comprising a transgenic human SLC25A12 gene. In some embodiments, these eukaryotic cells are particularly useful when the transgenic SLC25A12 gene comprises the sequence of SEQ ID NO:2 and/or SEQ ID NO:4, since those sequences are within SLC25A12 genes associated with autism.

In these embodiments, the cell is a yeast cell, or a mammalian cell, such as a brain cell. The cell can also be within a living mammal, such as a transgenic mammal transfected with the gene.

Thus, in related embodiments, the invention discloses non-human animals comprising the above described eukaryotic cells. Preferably, the non-human animal of these embodiments is a mammal.

Since it is likely that the SLC25A12 gene product is altered in autism, a mitochondrial aspartate/glutamate carrier (AGC1)(see Example 1), chemical compounds that affect AGC1 would be expected to affect autism.

Thus, the invention also discloses methods of evaluating whether a compound affects autism. The methods comprise contacting the compound with any of the above eukaryotic cells and determining whether the compound affects expression or activity of a product of the SLC25A12 gene. In these embodiments, a compound that affects expression or activity of the product of the SLC25A12 gene affects autism. The compounds useful for these embodiments can be a small organic or inorganic molecule or a macromolecule such as an antibody, an aptamer, an siRNA, an antisense compound, etc.

Preferred embodiments of the invention are described in the following examples.

### Example 1. Linkage and association of the mitochondrial aspartate/glutamate carrier AGC1/SLC25A12 gene with autism

### Example summary

Objective: Autism/autistic disorder (MIM #209850) is a complex, largely genetic psychiatric disorder. We recently mapped a susceptibility locus for autism to chromosome region 2q24-q33 (MIM #606053). In the present study, we analyzed genes across the 2q24-q33 interval to identify an autism susceptibility gene in this region.

Method: Mutation screening of positional candidate genes was performed in two stages. The first stage involved identifying genetic variants in exons and flanking sequence within candidate genes, in unrelated subjects showing linkage to 2q24-q33, and comparing the frequency of the variants between subjects and controls. Two single nucleotide polymorphisms (SNPs) that showed evidence for divergent distribution between subjects and controls were identified, both within SLC25A12, a gene encoding the mitochondrial aspartate/glutamate carrier (AGC1). In the second stage, the two SNPs in SLC25A12 were further genotyped in 411 autistic families, and linkage and association tests were carried out in the 197 informative families.

Results: Linkage and association were observed between autistic disorder and the two SNPs, rs2056202 and rs2292813, found in SLC25A12. Using a single affected per family, evidence for excess transmission was found by the transmission disequilibrium test (TDT) for rs2056202 (χ² =10.83, df=1, P=0.001), rs2292813 (χ²=6.23, df=1, P=0.01), and a two-locus G*G haplotype (χ²=22.10, df=1, P=0.000003). Using multiple affected individuals per family demonstrated evidence for linkage by the TDT for rs2056202 (χ² =8.89, df=1, P=0.003) and rs2292813 (χ²=7.28, df=1, P=0.007), and for the two-locus haplotype (χ²=20.41, df=1, P=0.000006). Evidence for linkage was supported by linkage analysis with the two SNPs, with a maximal multipoint NPL score of 1.64 and a maximal multipoint heterogeneity LOD score of 2.28.

Conclusions: Our studies demonstrated strong association of SNPs within the SLC25A12 gene with autism.

### Introduction

The aim of the present study was to identify an autism susceptibility gene in the 2q31 region. A systematic screen for genetic variants in affected individuals identified linkage and association between autism and single nucleotide polymorphisms in the SLC25A12 gene.

### Methods

Subjects. A total of four hundred eleven families were either recruited by the Seaver Autism Research Center (SARC) at Mount Sinai (n=40), co-recruited by SARC and the Autism Genetic Research Exchange (AGRE) (18) (n=127), or recruited by AGRE (n=244). All parents signed an informed consent and potentially affected individuals were assessed by the Autism Diagnostic Interview-Revised (ADI-R) (19). Individuals meeting ADI-R criteria for autism (19) or borderline autism (13) were defined as affected. The cohort (18) and research diagnosis definitions used in the current study (13) have been described. The entire cohort of 411 families included blood samples from more than 2000 individuals, including 720 affected individuals (671 with autism and 49 with borderline autism), and available parents and sibs. The 411 families included 274 multiplex (typically affected sibling-pairs) and 137 trio families. DNA from blood samples or transformed cells were either isolated as detailed in Buxbaum et al., 2001, or provided by the AGRE repository.

Mutation screening. To investigate the involvement of positional candidate genes in autism, we performed a two-stage screen.

In the first stage, exonic and flanking DNA from 35-47 patients from 38 autistic families linked to the chromosome 2q24-q33 region were screened for genetic variants by single strand conformation polymorphism (SSCP) and denaturing high-performance liquid chromatography (DHPLC). The rationale for this approach was to find variants in linked, affected individuals, rather then rely on variants in public databases identified in unaffected individuals, Furthermore, the focus was on exonic sequence and intronic sequence adjacent to exons, as being most likely to harbor functionally important variants.

Primers to amplify exons and flanking regions of positional candidate genes were designed using primer3 software (http://www-genome.wi.mit.edu/cgi-bin/primer/primer3_www.cgi) and are available on request. The forward and reverse primers were chosen to produce amplicons of less than 500 bp. Larger exons, in particular the last exons of the genes, were divided over several amplicons, each with a size of less than 500 base pairs. Primers within flanking introns were situated 26 to 186 bp from the exons. A total of 82 exons of 9 genes were analyzed, using 93 amplicons with an average size of ca. 310 nucleotides.

For SSCP, forward primers were labeled using 100 µCi [γP32]-ATP and 10 units of T4 polynucleotide kinase, according to the protocol provided by Invitrogen. Amplification was carried out using Ampli-Taq Gold Polymerase (Applied Biosystems) in a final volume of 10 µl, consisting of 10 ng of genomic DNA, 10 mM Tris-HCl, 50 mM KCl, 2.5mM MgCl, 100 µM dNTPs, 10 µM radioactively-labeled forward and unlabeled reverse primers, and 0.5 units of Taq Polymerase. Two µl of radioactively-labeled PCR product was then mixed with 2 µl of formamide blue loading buffer, denatured and separated on a non-denaturing MDE 0.5X gel (according to the protocol of BioWhittaker). SSCP were detected by autoradiography.

For DHPLC, 8 µl of PCR product was screened on the WAVE Nucleic Acid Fragment Analysis System (according to the manufacturer's protocols). Chromatographic parameters, appropriate analysis temperatures, and melting domains visualization were determined by WAVEMAKER software. Samples were using at least two mobile-phase temperatures to maximize the chances to identify polymorphisms.

Any amplicons in which variants were detected by either SSCP or DHPLC were then sequenced by direct fluorescent sequencing of purified PCR products using the BIG DYE dideoxy-terminator kit v3.1 (Applied Biosystems) and an ABI3100 DNA sequencer with Performance Optimized Polymer 6 (Applied Biosystems). Variants were then genotyped in 38 unrelated cases and in 100 controls. Allele frequencies were compared to identify variants with potentially altered frequencies (defined as P=0.1) between ethnically matched cases and controls (to reduce the chances for false-positives due to stratification).

In the second stage, variants with frequencies that were potentially altered between cases and controls were then analyzed in the entire cohort of more than 2000 individuals from 411 autistic families. Genotyping was carried out using the biplex DNA Invader method (Third Wave Technologies). The Invader assay is based on the hybridization of an oligonucleotide probe that completely matches a DNA target and the subsequent cleavage of the overlapping structure by Cleavase VIII, resulting in a target-specific product that is recognized by a fluorescence resonance energy transfer (FRET) cassette (20). Specific oligonucleotide probes corresponding to wild type and mutated SNPs are associated with specific fluorophores, enabling simultaneous detection of both DNA sequences in a single well. For our studies, diluted aliquots of the PCR products were combined with Invader mix solution (Third Wave Technologies), and incubated for the cleavage reaction in a thermal cycler (PTC-100, MJ Research). The reaction product was then analyzed on a fluorescence plate reader (CytoFluor Series 4000, PerSeptive Biosystems) using the appropriate parameters of excitation and emission for each fluorophore.

Statistical analysis. Two-tailed chi-square tests (χ²) were used for comparisons of allelic frequencies and distributions of genotypes between control and autism groups. Fischer's exact test was performed when the number in a group was less than five. Hardy-Weinberg distribution was examined for each identified polymorphism in autistic and control groups.

Statistical analyses for transmission disequilibrium tests (TDT) were computed with TDT-GENEHUNTER (GENEHUNTER version 2.1, compiled to run on the Unix environment of Mac OS X) and the S-TDT (21) program (http://genomics.med.upenn.edu/spielman/TDT.htm). Two-locus TDT was carried out with TDT-GENEHUNTER using the TDT2 option and the haplotypes were constructed by GENEHUNTER and verified manually to ensure structure. Haplotypes were determined on the basis of transmission patterns in families in which both parents were genotyped.

For linkage analysis, we used GENEHUNTER PLUS (compiled to run on the Unix environment of Mac OS X). Scores for heterogeneity LOD (HLOD) and nonparametric linkage (NPL) were calculated for both single and multi-point analyses. For HLOD, data were analyzed under both a dominant and recessive model, using 50% penetrance and a value of 0.001 for the disease allele frequency. Such an approach detects linkage under many different conditions irrespective of the "true" underlying inheritance pattern (22, 23).

Linkage disequilibrium (LD) was estimated using a D' value calculated with the 2LD program (24).

### Results

Genes across the 2q31 region were screened for association in two stages as detailed in the Methods. The genes analyzed included glutamate decarboxylase 1 (GAD1) (in collaboration with Drs. Shigeo Kure, Kiyoshi Kanno and Yoichi Matsubara), four hypothetical proteins (FLJ13096, FLJ13984, LOC130672, and FLJ23462, recently identified as duodenal cytochrome b) (in collaboration with Drs. Paolo Gasparini and Massimo Carella), histone acetylase-1 (HAT-1) (in collaboration with Dr. Salah Uddin Qureshi), the cytoplasmic dynein subunit DNCI2, the asparate/glutamate carrier SLC25A12, and the homeobox protein DLX2 (Figure 1C). These candidate genes were chosen based on their position relative to the positive linkage results from three studies (13-15), their expression in brain tissue, and, in some cases, their known function, their novelty, or the existence of related genes within the region of chromosome 7 showing linkage to autism. For this latter criterion, we note that the linked region of chromosome 7 contains genes paralogous to DNCI2, SLC25A12, DLX1 and DLX2 (i.e., DNCI1, SLC25A13, DLX5 and DLX6).

In the first stage, all known exons (with flanking intronic sequence) of these genes were screened by SSCP and DHPLC for variants in 35 to 47 unrelated individuals chosen from families showing linkage to D2S335 as described in Methods. In the nine genes, 82 exons were screened and 29 SNPs were identified. Frequencies of each variant were then evaluated in the autistic patients (using only one affected individual per family, n=38) and in 50 ethnically matched controls, after confirming that the distribution of allele frequencies were in Hardy-Weinberg equilibrium. Only two SNPs, both within the SLC25A12 gene, showed significant differences in allele frequencies between cases and controls using both allele- (P<0.004) and genotype-based (P<0.03) tests.

Within the SLC25A12 gene, we identified a total of five variants in the first stage screen (including the two meeting criteria for further study, indicated above). Figure 1C presents the five variants of the SLC25A12 gene identified in 47 affected subjects linked to the chromosome 2q24-q33 region. The two polymorphism meeting criteria in the first stage, rs2056202 (I3-21A/G) and rs2292813 (I16+70A/G), are G/A variants in flanking intronic sequence located 21 bp upstream of exon 4 and 70 bp downstream of exon 16, respectively. Two variants, C->T at nucleotide 99 (rs1878583) and G->A at nucleotide 1418, were within coding regions. G1418A changes arginine 473 to glutamine, while the C99T variant is silent. G1418A is a new SNP, not reported in the NCBI dbSNPs database, located in a region conserved across mammalian species, but the amino acid glutamine is observed in mice. The final variant appears in the 3' untranslated region (UTR). We did not find SNP rs 1059299, reported in the public database, which changes amino acid 600, in our sample.

Given the evidence for association of rs2056202 and rs2292813 in a small number of cases and controls, the entire sample was genotyped at these SNPs for analysis by the Transmission Disequilibrium Test (TDT), which makes use of family-based controls. Of the 411 families studied, 197 had at least one parent heterozygous for at least one SNP. These families included 140 multiplex and 57 singleton families. To test for association by the TDT, transmission from heterozygous parents to one affected child was analyzed (Table 1). TDT analysis demonstrated association for rs2056202 (χ²=10.83, df=1, P=0.001) and for rs2292813 (χ²=6.23, df=1, P=0.01). In both cases, the G allele appeared to be the risk allele (or the A allele the protective allele) (for simplicity, Table 1 and 2 show transmission data for just the G allele for both SNPs).

**Table 1. TDT with one affected per family**

| | T | NT | χ² | P | T | NT | χ² | P | T | NT | χ² | P |
|---|---|---|---|---|---|---|---|---|---|---|---|---|
| | Combined | | | | Maternal | | | | Paternal | | | |
| **SNPs** | | | | | | | | | | | | |
| rs2056202 | 116 | 71 | 10.83 | 0.001 | 53 | 30 | 6.37 | 0,01 | 53 | 31 | 5.76 | 0.02 |
| rs2292813 | 72 | 45 | 6.23 | 0.01 | 40 | 19 | 7.47 | 0.006 | 27 | 21 | 0.75 | 0.39 |
| **Haplotypes** | | | | | | | | | | | | |
| G*G | 102 | 45 | 22.10 | 3X10⁻ | 54 | 19 | 66.52 | 3X10⁻⁶ | 48 | 26 | 11.92 | 6X10⁻⁴ |
| G*A | 8 | 9 | 0.06 | 0.81 | 3 | 8 | 2.9 | 0.09 | 5 | 1 | 3 | 0.08 |
| A*G | 21 | 47 | 9.94 | 0.002 | 11 | 23 | 7.11 | 0.008 | 10 | 24 | 9.84 | 0.002 |
| A*A | 26 | 56 | 10.98 | 0.001 | 12 | 30 | 13.76 | 0.0002 | 14 | 26 | 6.05 | 0.014 |

| | | | | | | | | | | | | |
|---|---|---|---|---|---|---|---|---|---|---|---|---|
| For each SNP or haplotype, the number of transmitted (T) or non-transmitted (NT) events are shown. For the individual SNPs, data is the for G allele. | | | | | | | | | | | | |

TDT analysis was also carried out using multiple affected individuals per family (Table 2). Such analysis is more properly a measure of linkage rather than association. Transmission disequilibrium was observed for both rs2056202 (χ²=8.89, df=1, P=0.003) and rs2292813 (χ²=7.28, df=1, P=0.007).

**Table 2. TDT with all affecteds per family**

| | T | NT | χ² | P |
|---|---|---|---|---|
| SNPs | | | | |
| rs2056202 | 191 | 137 | 8.89 | 0.003 |
| rs2292813 | 124 | 85 | 7.28 | 0.007 |
| Haplotype | | | | |
| G*G | 163 | 91 | 20.41 | 6X10⁻⁶ |
| G*A | 11 | 16 | 0.93 | 0.34 |
| A*G | 44 | 71 | 6.34 | 0.011 |
| A*A | 55 | 95 | 10.67 | 0.001 |

| | | | | |
|---|---|---|---|---|
| For each SNP or haplotype, the number of transmitted (T) or non-transmitted (NT) events are shown. For the individual SNPs, data is for the G allele. | | | | |

Looking at haplotypes, there was an increased transmission of the G*G haplotype in autism when analyzing either one affected per family (χ²=22.10, df=1, P=0.000003) or all affecteds (χ²=20.41, df=1, P=0.0000006). Using a global analysis, two-locus TDT showed disequilibrium of transmission of the four observed haplotypes for both one affected per family (χ²=32.31, df=3, P=5X10⁻⁷) or all affecteds (χ²=28.76, df=3, P=0.000003).

Two-point linkage analysis using nonparametric LOD score analysis (NPL), indicated some evidence for linkage between autism and rs2056202 (NPL=1.26, P=0.07) or rs2292813 (NPL=1.1, P=0.09) (Table 3). Two-point heterogeneity LOD (HLOD) supported this linkage, with maximal HLOD scores of 2.11 (P=0.03) and 1.15 (P=0.1), for rs2056202 and rs2292813, respectively. However, information was low at these SNPs (estimated as 0.23 and 0.34 for rs2056202 and rs2292813, respectively). To increase information we used multipoint linkage analyses with these two SNPs. Under these conditions, maximal multipoint NPL scores of 1.57 and maximal multipoint HLOD scores of 2.11 were observed (information was increased to about 0.51). The two markers showed linkage disequilibrium with each other as determined by analyzing linkage disequilibrium in unrelated patients (D'=0.79, SD=0.06).

**Table 3. Linkage analysis of SNPs in SLC25A12**

| **Two-point linkage analysis** | | |
|---|---|---|
| | rs2056202 | rs2292813 |
| NPL | Z score=1.26; P=0.07 | Z score=1.11; P=0.09 |
| HLOD, recessive model | HLOD=1.52; P=0.06; α=0.21 | HLOD=1.79; P=0.04; α=0.28 |
| HLOD, dominant model | HLOD=0.75; P=0.19; α=0.29 | HLOD=0.8; P=0.18; α=0.39 |

| **Multipoint linkage analysis** | | |
|---|---|---|
| NPL | Maximal Z score=1.57; P=0.03 | |
| HLOD, recessive model | Maximal HLOD=2.11; P=0.03; α=0.23 | |
| HLOD, dominant model | Maximal HLOD=1.15; P=0.1; α=0.34 | |

| | | |
|---|---|---|
| P values for HLOD scores were computed as described in Nyholt 2000 (36). α is an estimate of the fraction of families showing linkage at the locus under study. | | |

A genotype relative risk was also estimated for individuals carrying one or two copies of the risk alleles (the G alleles in both cases) for either rs2056202 or rs2292813. Results are given in FIG. 2. Those results show that the relative risk increases with increasing G alleles at either polymorphic site. It should also be noted that genotype relative risk estimates in TDT studies such as this underestimate the true genotype relative risk. See Risch, Theoret. Pop. Biol. 60, 215-220 (2001). Therefore, the true autism risk for the genotypes indicated in FIG. 2 is likely to be considerably higher than indicated.

### Discussion

The objective of the present work was to identify a susceptibility gene for autism in chromosome region 2q24-q33. We identified two SNPs, rs2056202 and rs2292813, in SLC25A12 that demonstrated association with autism using the TDT. Preferential transmission of the G allele for both SNPs was found in 197 informative families. Furthermore, linkage was found between autism and the SNPs by TDT and by non-parametric and parametric analyses.

The SLC25A12 gene contains 18 exons spreading over about 110 kilobases (kb) and is expressed primarily as 2.9 and 3.2kb mRNA species, predominantly in skeletal muscle, heart and brain (25). SLC25A12 cDNA has an open reading frame of 2037 bp encoding a 678 amino acids protein that is a calcium-dependent mitochondrial aspartate/glutamate carrier (AGC 1). The subcellular localization of protein is exclusively mitochondrial. The amino-terminal half of AGC1 contains five putative EF hands that are able to bind Ca2+, while the carboxy-terminal half harbors the aspartate/glutamate exchanger function. AGC1 is critically involved in the activity of the malate/aspartate NADH shuttle, catalyzing the electrogenic exchange of aspartate for glutamate and a proton, as well as in the urea cycle (26). Recently, it has been shown that the SLC25A12 gene, as well as AGC1, is the only form of the mitochondrial aspartate/glutainate carrier expressed in neurons and neural stem cells (27). Protein levels increased during neuronal differentiation and are correlated with an increase in the malate/aspartate NADH shuttle activity.

SLC25A12 mRNA and the AGC1 protein are widely expressed in adult mouse CNS, particularly in neural nuclei in the brainstem (27). It has been suggested that the enrichments of AGC1 in specific neurons could reflect a tonic activity of these neurons. Dysfunction of this protein, or altered expression of this protein, may lead to an alteration in mitochondrial function and ATP synthesis. As neurons are major energy users, even modest changes in mitochondrial function and ATP synthesis may lead to selective changes in neurons. Support for a role for mitochondrial dysfunction in autism comes from a recent study demonstrating mitochondrial hyperproliferation and partial respiratory chain block in two patients with autism and a 15q inverted duplication (28).

As noted above, a region of chromosome 7 has also been shown to be linked to autism in multiple studies (29). It is interesting that a paralog of SLC25A12, namely SLC25A13 (CITRIC1 or AGC2), localizes to this region of chromosome 7. These two genes share about 79% identity, both encoding forms of aspartate/glutamate carriers, with 71% identity in the EF-hand domains and 84% within the exchanger domain. Mutations in SLC25A13 gene confers adult-onset type II citrullinemia (CTLN2, MIM#603471), an autosomal recessive disease caused by a deficiency of argino-succinate synthetase with clinical features included enuresis diarrhea, tremors, lethargy, mental retardation and psychiatric disorders. Although confirmation of the association between autism and SLC25A12 is required, it is tempting to speculate that SLC25A13 could be a candidate autism susceptibility gene on chromosome 7.

For both SNPs, the allele associated with autism corresponds to a common allele. One must consider that in complex disorders with multiple interacting genes, the prevalence of the susceptibility alleles may be quite high. Recent examples of this include the e4 allele of apoliprotein E in Alzheimer' s disease, NOD-2 gene changes in Crohn's disease, and calpain-10 gene changes and type 2 diabetes mellitus (30-32). In the case of autism, with the strongest evidence for numerous interacting genes, the expectation would be that the susceptibility variants for at least some loci will be quite common, and might even contribute to behavioral variability in healthy individuals. However, in any such study the true susceptibility locus or functional polymorphism may not have been identified but rather are in linkage disequilibrium with the variants studied. The true susceptibility locus may even be in neighboring genes. In our studies, we have examined both flanking genes. For HAT1, we did not find any useful polymorphisms. For DNCI2, we found 8 polymorphisms that were negative for association in first stage analyses.

In our studies, we identified intronic polymorphisms associated with disease. The functional relevance of such variants remains obscure in most studies. However, it is increasingly being realized that the expression of a significant number of genes is regulated by cis-acting elements and that inherited variation in gene expression may contribute to disease (33, 34). Regulation of gene expression would affect cellular function, without requiring a modification in the coding sequence, if levels of the gene product were limiting. It has recently been demonstrated that over-expression of AGC1 can lead to increased mitochondrial ATP production (35), so genetic variations that change the expression of AGC1 would be predicted to impact on ATP production. Whether the polymorphisms we identified (or additional polymorphism in linkage disequilibrium with the polymorphism we identified) affect gene expression needs to be determined.

With all association studies, especially in complex disorders thought to be due to multiple interacting genes of weak effect, we must await replication in independent samples before the results can be accepted. However, given the evidence from our study, it may be unrealistic to expect that the finding will be easily replicated by TDT in a typical sample of under 200 trios. The TDT allows for robust statistical analysis without bias of population admixture. However, it lacks power, especially at loci such as ours, where many of the parents were homozygous at the two loci. A carefully designed case-control study, with controls matched for ethnicity, gender, and age, may have more power to detect association at these two loci. Alternatively, genotyping several hundred trios for TDT would be in order for a replication study.

Assuming that our results reflect true association of SLC25A12 with autism, the data indicate a genotype relative risk for the two-loci of between 2 and 5 (FIG. 2). This, while significant, must be taken in context of the observation that the susceptibility variants (or the variants in linkage disequilibrium with the true susceptibility variants) are common alleles. This is consistent with the idea that this locus plays a significant role in the epidemiology of the disorder but would not be immediately useful for genetic counseling, until it can be considered together with additional loci.

To further investigate SLC25A12 locus as a susceptibility gene for autism we are currently searching for additional genetic markers across the 110 kb region containing this gene, using both extant databases and sequencing in the patients we are studying, particularly of conserved non-coding regions. To date, more than 90 SNPs encompassing the SLC25A12 gene have been identified in the public databases, with at least 20 SNPs harboring a heterozygosity rate over 0.1. None of these appear in conserved non-coding regions. We are also carrying out expression studies of AGC1.

### Example 2. Further association of AGC1/SLC25A12 with autism.

In Example 1, we reported the linkage and association between autism and the presence of two single nucleotide polymorphisms (SNPs), both within the same gene, SLC25A12/AGC1, an aspartate/glutamate exchanger. We have screened 12 additional SNPs, covering the entire 110 kbps of the SLC25A12/AGC1 gene, in 360 families. All of these SNPs harbor significant p values for multipoint non-parametric lod score analysis. This observation confirms the linkage between autism and SLC25A12/AGC1 gene. In this sample, as reporter earlier, association tests (GH-TDT: Transmission Disequilibrium Test by Genehunter for all affecteds, and Transmit for all affecteds or one random affected) are positive for rs2292813 (GH-TDT: Chi2=4.79, p=0.03; Transmit-all: Chi2=4.27, p=0.04) and rs2056202 (GH-TDT: Chi2=8.5, p=0.004; Transmit-all: Chi2=8.73, p=0.003; Transmit one: Chi2=7.16, p=0.014; TDT-Transmit one: Chi2=7.29, p=0.01).

In the additional SNPs screened, we also observed an association between autism and hCV1735157 (Transmit one: Chi2=5.3, p.018; TDT-Transmit one: Chi2=5.43, p=0.02).

Combinations of two SNPs, or haplotypes, also give positive associations. We noted that a G*rs2292813-T*hCV1735157 haplotype is associated in the families (1). Similarly, two-locus haplotypes were positive for T*rs925881-G*rs2056202 (2) and G*rs2056202-C*rs 1996425 (3). Positive p values for global tests strongly support association between autism and several haplotypes.

In conclusion, among the 12 additional SNPs genotyped in 360 families, we have evidence, in addition to rs2292813 and rs2056202, for linkage and associations between autism and hCV1735157, rs925881 and rs1996425, which are all SNPs across the SLC25A12/AGC1 gene.
(1) GH-TDT: Transmitted=95, Not Transmitted=69, Cho2=4.12, p=0.04; Global test for all haplotypes: Chi2=8.81, p=0.032.
(2) GH-TDT: Transmitted=142, Not Transmitted=101, Chi2=6.92, p=0.009; Global test for all haplotypes: Chi2=16.01, p=0.001; Transmit-all: Chi2=4.04, p=0.044.
(3) GH-TDT: Transmitted=139, Not Transmitted=100, Chi2=6.36, p=0.01; Global test for all haplotypes: Chi2=16.19, p=0.001; Transmit-all: Chi2=4.23, p=0.04; TDT-Transmit one: Global test for all haplotypes: Chi2=8.4, p=0.038.

The discussion of the references herein is intended merely to summarize the assertions made by the authors and no admission is made that any reference constitutes prior art. Applicants reserve the right to challenge the accuracy and pertinence of the cited references.

### Appendix - SEQ ID Nos

SEQ 1D NO:1 region around rs2056262 that is amplified with SEQ ID NO:5 and SEQ ID NO:6 - with A form; the A at the polymorphic site is underlined 
GTTACCCTGAGCTACAGTTATATAGTAAAGGCAGATTAAATGCCTGATGCTTTCCCT
TTCTTCACCAGCCAATGACGTTTATATTTATTCCAGGTTGATCTCCTATCAAGAGTT
TTTGGCATTTGAATCTGTTTATGTGCTCCAGATTCCATGTTCATAGTGGCTTTCCAGT
TGTTTGACAAGAGTGGAAATGGAGAGGTGACATTTGGTAAGGGAAAAAGAAGATTA
TAAGTGATAAGTTAATGATGCTGGTCCAGTCTTCAATTGCTGAATCTAGTAACTAATA
TAGATTACTGCTTATTTGGGATCTGA
SEQ ID NO:2 region around rs2056262 that is amplified with SEQ ID NO:5 and SEQ ID NO:6 - with G form; the G at the polymorphic site is underlined 
GTTACCCTGAGCTACAGTTTATATAGTAAAGGCAGATTAAATGCCTGATGCTTTCCCT
TTCTTCACCAGCCAATGGCGTTTATATTTATTCCAGGTTGATCTCCTATCAAGAGTT
TTTGGCATTTGAATCTGTTTATGTGCTCCAGATTCCATGTTCATAGTGGCTTTCCAGT
TGTTTGACAAGAGTGGAAATGGAGAGGTGACATTTGGTAAGGGAAAAAGAAGATTA
TAAGTGATAAGTTAATGATGCTGGTCCAGTCTTCAATTGCTGAATCTAGTAACTAATA
TAGATTACTGCTTATTTGGGATCTGA
SEQ ID NO:3 region around rs2292813 that is amplified with SEQ ID NO:7 and SEQ ID NO:8 - with A form; the A at the polymorphic site is underlined 
CCGCTCAAGTGGTTGAAGTTGCAGGCTCTATAGTAGATGTTAGCAGTATTCCTATCTT
TTATAAGGTACTCTAGATAAATTAAATGTGGTTTCTCCTGAAAGGTGTCCCAGCTGC
ATCTCTGGTGACCCCTGCTGATGTCATCAAGACAAGACTGCAGGTGGCTGCCCGCGC
TGGCCAGACGACATACAGTGGTGTCATCGACTGTTTCAGGAAGATTCTCCGGGAAGA
AGGGCCCTCAGCATTTTGGAAAGGGACTGCAGGTAGGCAGGGGCTGGAGCCATACA
GAATGGCTGGCTGGCTCTAGCGTCCTCCCCTGTGACTCAGTGGCTATCTTTACCACAT
TTGTCCTGGTTTCAAGTCTCCCCTGCCCCTGCTTCTCTTTTCAGCTCGAGTGTTTCGA
TCCTCTCCCCAGTTTGGTGTTACCTTGGTCACTTATGAACTTCTCCAGCGGTGGTTTTA
CATTGATTTTGGAGGCCTGTAAGTCAGCTGCTCAACTCCTTTACAAAGAAATCACTAA
GTCCAAAACAAATGTTTGTTCTGTCTACAAAAGCATTGTTGCAACTCTTAGAAAACTG
ATAAGACAGAACCTTTAAGACCAATGC
SEQ ID NO:4 region around rs2292813 that is amplified with SEQ ID NO:7 and SEQ ID NO:8 - with G form; the G at the polymorphic site is underlined 
CCGCTCAAGTGGTTGAAGTTGCAGGCTCTATAGTAGATGTTAGCAGTATTCCTATCTT
TTATAAGGTACTCTAGATAAATTAAATGTGGTTTCTCCTGAAAGGTGTCCCAGCTGC
ATCTCTGGTGACCCCTGCTGATGTCATCAAGACAAGACTGCAGGTGGCTGCCCGCGC
TGGCCAGACGACATACAGTGGTGTCATCGACTGTTTCAGGAAGATTCTCCGGGAAGA
AGGGCCCTCAGCATTTTGGAAAGGGACTGCAGGTAGGCAGGGGCTGGAGCCATACA
GAATGGCTGGCTGGCTCTAGCGTCCTCCCCTGTGACTCAGTGGCTGTCTTTACCACAT
TTGTCCTGGTTTCAAGTCTCCCCTGCCCCTGCTTCTCTTTTCAGCTCGAGTGTTTCGA
TCCTCTCCCCAGTTTGGTGTTACCTTGGTCACTTATGAACTTCTCCAGCGGTGGTTTTA
CATTGATTTTGGAGGCCTGTAAGTCAGCTGCTCAACTCCTTTACAAAGAAATCACTAA
GTCCAAAACAAATGTTTGTTCTGTCTACAAAAGCATTGTTGCAACTCTTAGAAAACTG
ATAAGACAGAACCTTTAAGACCAATGC
SEQ ID NO:5 forward primer for amplifying rs2056202
   GTTACCCTGAGCTACAGTT
SEQ ID NO:6 reverse primer for amplifying rs2056202
   TCAGATCCCAAATAAGCAG
SEQ ID NO:7 forward primer for amplifying rs2292813
   CCGCTCAAGTGGTTGAAGTT
SEQ ID NO:8 reverse primer for amplifying rs2292813
   GCATTGGTCTTAAAGGTTCTGTCT

## Claims

1. A method of evaluating an individual for relative genetic risk for autism, the method comprising determining the individual's genotype at polymorphism sites rs2056202 and/or rs2292813 of the SLC25A12 gene, wherein the presence of a G at either of the two sites indicates an increased risk for autism.

2. The method of claim 1, wherein the genotype are determined by one or more methods selected from the group consisting of single strand conformation polymorphism, denaturing high-performance liquid chromatography, DNA Invader, and polymerase chain reaction amplification followed by sequencing.

3. The method of claim 1, using polymerase chain reaction amplification with at least one primer comprising a sequence selected from the group consisting of SEQ ID NO:5, SEQ ID NO:6, SEQ ID NO:7, and SEQ ID NO:8.

## Patentansprüche

1. Ein Verfahren zur Untersuchung eines Individuums auf ein relatives genetisches Risiko für Autismus, wobei das Verfahren die Bestimmung des Genotyps des Individuums and den Polymorphismusorten rs2056202 und/oder rs2292813 des SLC25A12-Gens umfasst, wobei die Anwesenheit eines G an einer der beiden Stellen ein erhöhtes Risiko für Autismus indiziert.

2. Das Verfahren nach Anspruch 1, wobei der Genotyp durch ein oder mehrere Verfahren ausgewählt aus der Gruppe bestehend aus Einzelstrang-Konformations-Polymorphismus, denaturierende Hochleistungsflüssigkeitschromatographie, DNA Invader Assay und Polymerase-Kettenreaktion-Amplifikation gefolgt von einer Sequenzierung.

3. Das Verfahren nach Anspruch 1, unter Verwendung der Polymerase-Kettenreaktion-Amplifikation mit mindestens einem Primer umfassend einer Sequenz ausgewählt aus der Gruppe bestehend aus SEQ ID NO: 5, SEQ ID NO:6, SEQ ID NO:7 und SEQ ID NO:8.

## Revendications

1. Méthode d'évaluation chez un individu du risque génétique relatif pour l'autisme, la méthode comprenant la détermination du génotype de l'individu aux sites polymorphiques rs2056202 et/ou rs2292813 du gène SLC25A12, dans laquelle la présence d'un G à l'un ou l'autre des deux sites indique un risque accru pour l'autisme.

2. Méthode de la revendication 1, dans laquelle le génotype est déterminé par une ou plusieurs méthodes sélectionnées dans le groupe consistant en le polymorphisme de conformation simple brin, la chromatographie liquide haute performance dénaturante, l'Invader ADN, et l'amplification en chaine par polymérase suivie par un séquençage.

3. Méthode de la revendication 1, utilisant l'amplification en chaine par polymérase avec au moins une amorce comprenant une séquence sélectionnée dans le groupe consistant en SEQ ID NO: 5, SEQ ID NO: 6, SEQ ID NO: 7, et SEQ ID NO: 8.
